# FASCICULE DE BREVET EUROPEEN

(11) **EP 0 692 239 B1**
(45) Date de publication et mention de la délivrance du brevet: **25.09.1996**
(21) Numéro de dépôt: 95401267.0
(22) Date de dépôt: 31.05.1995
(51) Int. Cl.: A61K 7/00, A61K 7/46

(54) **Composition liquide parfumante biphasique**
Flüssige, zweiphasige parfümierende Zusammensetzung
Liquid biphasic perfuming composition

(30) Priorité: 06.06.1994 FR 9406898
(43) Date de publication de la demande: 17.01.1996
(73) Titulaire: L'OREAL, F-75008 Paris (FR)
(72) Inventeur: Ser, Jocelyne, F-94550 Chevilly-Larue (FR)
(74) Mandataire: Lhoste, Catherine

(56) Documents cités:
- EP-A- 0 370 856
- EP-A- 0 603 080
- DE-A- 4 241 799
- GB-A- 2 206 048

## Description

L'invention a pour objet une composition liquide parfumante à deux phases distinctes, destinée en particulier à remplacer les eaux de toilette.

Les compositions parfumantes utilisées jusqu'à présent, telles que les eaux de toilette et les produits apparentés comme les eaux fraîches, les eaux de parfum et les lotions après-rasage, se présentent actuellement sous forme de compositions liquides homogènes transparentes constituées d'une phase hydroalcoolique contenant au moins une substance aromatique qui leur donne leur odeur.

Il est important que ces produits se présentent sous forme de compositions transparentes pour donner à l'utilisateur un aspect de fraîcheur rien qu'à la vue du produit. D'autre part, pour que l'utilisateur garde l'odeur du parfum un certain temps sur lui, ces produits doivent contenir une quantité suffisante de substance aromatique. Afin de solubiliser la substance aromatique, il est nécessaire d'utiliser une certaine quantité d'alcool, et les eaux de toilette ont par conséquent un degré d'alcool important, généralement supérieur à 80°. De ce fait, les compositions parfumantes classiques présentent l'inconvénient d'être parfois irritantes en raison de leur degré d'alcool élevé.

En vue de diminuer le caractère irritant de ces compositions, on peut envisager d'abaisser le degré d'alcool. Malheureusement, dans ce cas, il faut aussi diminuer le taux de substance aromatique. Il s'ensuit que l'eau de toilette obtenue est beaucoup moins parfumante.

Par ailleurs, il est connu de faire des compositions parfumées contenant une phase grasse, par exemple des laits hydratants pour le corps. Malheureusement, ces compositions ne sont pas transparentes et ne répondent donc pas aux critères habituels des eaux de toilette. D'autre part, elles ont un caractère parfumant moindre que celles des eaux de toilette. Ce sont des produits différents des eaux de toilette ; leur but d'utilisation étant avant tout de traiter la peau tout en lui conférant une légère odeur agréable.

Il subsiste donc le besoin d'une composition liquide transparente parfumante ayant un degré d'alcool inférieur à celui des eaux de toilette classiques, tout en gardant un bon pouvoir parfumant.

La composition selon l'invention permet d'atteindre ce but. La demanderesse a en effet trouvé de manière surprenante qu'il était possible d'obtenir une composition parfumante non irritante, ayant un parfumage équivalent à celui des eaux de toilette classiques en utilisant une composition à deux phases comprenant deux phases non miscibles.

L'invention a donc pour objet une composition liquide parfumante, caractérisée en ce qu'elle comporte deux phases distinctes, une phase hydroalcoolique contenant de 40 à 90 % d'éthanol et au moins une substance aromatique, et une phase huileuse contenant au moins une huile silicone volatile.

La présence d'une phase huileuse associée à la phase aqueuse permet d'abaisser le taux d'alcool et donc d'amener un plus grand confort lors de l'utilisation ainsi qu'une irritation plus faible. En outre, il est surprenant que la composition selon l'invention conserve un pouvoir parfumant aussi élevé que celui des eaux de toilette connues.

L'invention proposée est donc un nouveau concept de produit parfumant biphasique à agiter avant l'emploi, ayant un aspect esthétique attrayant. En particulier, les deux phases de la composition sont transparentes.

L'invention a donc encore pour objet l'utilisation de la composition telle que définie ci-dessus comme eau de toilette et/ou comme lotion après-rasage.

On connaît des compositions cosmétiques liquides à deux phases, notamment des compositions de nettoyage ou de soin de la peau, contenant une phase huileuse et une phase aqueuse se trouvant au repos l'une au-dessus de l'autre. Au moment de l'emploi, on agite les compositions pour obtenir une dispersion d'une phase dans l'autre. Avec ce type de composition, il est important d'obtenir la formation d'une dispersion homogène par secouage, puis un retour rapide à sa forme initiale biphasique.

L'homogénéité de la dispersion est généralement obtenue grâce à la présence d'émulsionnants. Une composition biphasique de nettoyage contenant un émulsionnant est notamment décrite dans le document FR-A-2254636. On peut aussi homogénéiser la dispersion en utilisant des particules au lieu d'émulsionnant, comme c'est décrit par exemple dans le document FR-A-2208642 qui se rapporte à une composition de soin. Par ailleurs, le document EP-A-494391 décrit une composition cosmétique de soin à deux phases transparentes, dont la transparence des phases est obtenue grâce à la présence d'un monoester d'acide gras de triglycérol.

L'invention présente l'avantage non seulement de ne contenir aucun émulsionnant ni particules stabilisantes, mais encore d'avoir une teneur en alcool plus faible que celle utilisée dans les eaux de toilette classiques.

Généralement, l'alcool utilisé est l'alcool éthylique qui est le solvant habituel des eaux de toilette.

De façon avantageuse, le degré d'alcool de la composition selon l'invention est inférieure à 60° et mieux à 50°. En pratique, le degré d'alcool peut notamment aller de 40° à 50°.

De préférence, la phase hydroalcoolique peut représenter de 80 % à 20 % en poids par rapport au poids total de la composition, et mieux de 70 % à 50% en poids.

En pratique, l'alcool peut représenter de préférence de 40 % à 90 % en poids de la phase hydroalcoolique, et mieux de 60 à 75 % en poids.

Par ailleurs, la phase huileuse peut représenter de préférence de 20 % à 80 % en poids par rapport au poids total de la composition, et mieux de 30 % à 50 % en poids.

Selon l'invention, la phase huileuse contient au moins une huile de silicone volatile. On peut citer par exemple comme huiles de silicone volatiles utilisables dans l'invention les cyclométhicones, telles que celle vendue sous la dénomination Volatile Silicone 7207 par la société Union Carbide, et les diméthicones, telles que celle vendue sous la dénomination DC 200 Fluid par la société Dow Corning. L'huile de silicone volatile représente de 30 % à 100 % de la phase huileuse, de préférence de 60 % à 95 %, et mieux de 70 % à 80 %.

La phase huileuse peut contenir aussi d'autres corps gras, notamment des esters gras (myristate d'isopropyle), des huiles minérales (vaseline), des huiles végétales (huile de tournesol), des huiles de synthèse (squalane), des triglycérides d'acides gras (triglycérides d'acides caprylique/caprique), des huiles de silicone non volatiles. Par ailleurs, elle peut contenir des adjuvants liposolubles comme des colorants et des actifs tels que les vitamines liposolubles ou l'alpha-bisabolol.

De façon avantageuse, la phase hydroalcoolique, en plus de l'alcool et de la ou des substances aromatiques, peut contenir des glycols tels que la glycérine, les polyéthylène glycols (polyéthylène glycol 400), le propylène glycol, le butylène glycol, le dipropylène glycol et l'hexylène glycol. Ces glycols peuvent être présents à un taux allant jusqu'à 35 % en poids par rapport au poids total de la composition, et de préférence à un taux allant de 10 % à 20% en poids.

La phase hydroalcoolique peut, en outre, contenir d'autres adjuvants hydrosolubles tels que des sels minéraux comme le chlorure de sodium, des colorants, des actifs hydrosolubles tels que le D-panthénol et l'extrait glycolique de ginseng.

Les adjuvants hydrosolubles ou liposolubles peuvent être présents à un taux allant jusqu'à 10 % en poids par rapport au poids total de la composition, et de préférence à un taux allant de 0,5 % à 2 % en poids.

La substance aromatique constitue le parfum de la composition et peut être formée d'un mélange d'huiles essentielles telles que *Litsea Cubeba*, le bois de rose, le coriandre russe, l'orange, le géranium et la myrthe, ou d'un mélange de substances odorantes telles que les alcools aliphatiques et aromatiques, les aldéhydes aliphatiques et aromatiques, les cétones. Ce peut notamment être un parfum. La ou les substances aromatiques peuvent représenter de 0,3 % à 15 % en poids de la composition, et mieux de 1 % à 5 % en poids.

Les exemples suivants illustrent l'invention. Dans ces exemples, les proportions indiquées représentent des pourcentages en poids.

### Exemple 1: Lotion biphasique parfumante pour le corps (eau de toilette)

| *Phase aqueuse :* | |
|---|---|
| Alcool éthylique à 96° | 40,0 |
| Polyéthylène glycol 400 | 5,0 |
| Glycérine | 5,0 |
| Parfum (Concentré PLB 430/5 vendu par la société Takasago) | 5,0 |
| Chlorure de sodium | 0,1 |
| Colorant | qs |
| Eau déminéralisée | 4,9 |

| *Phase huileuse :* | |
|---|---|
| Cyclométhicone (Volatile Silicone 7207 de la société Union Carbide) | 29,0 |
| Myristate d'isopropyle | 10,0 |
| Huile de tournesol | 1,0 |
| Colorant | qs |

La préparation de la lotion est réalisée en deux temps :
- On dissout d'abord le chloure de sodium dans l'eau, puis on y ajoute dans l'ordre les autres ingrédients de la phase aqueuse. On refroidit la phase hydroalcoolique obtenue aux environs de -5°C et on la filtre sur filtre-presse à cette température.
- On mélange par ailleurs tous les ingrédients de la phase huileuse dans l'ordre en agitant faiblement.
- On additionne ensuite les deux phases à la température ambiante pour obtenir la lotion biphasique.

Il faut agiter la lotion avant chaque utilisation.

### Exemple 2: Lotion biphasique après-rasage

| *Phase aqueuse :* | |
|---|---|
| Alcool éthylique à 96° | 50,0 |
| Polyéthylène glycol 400 | 5,0 |
| Propylène glycol | 5,0 |
| Parfum (Concentré FAN 62535 vendu par la société IFF) | 1,0 |
| Chlorure de sodium | 0,1 |
| D-panthénol | 0,2 |
| Eau déminéralisée | 8,7 |

| *Phase huileuse :* | |
|---|---|
| Cyclométhicone (DC 200 Fluid de la société Dow Corning) | 21,8 |
| Alphabisabolol | 0,2 |
| Triglycérides d'acides caprylique/caprique (Miglyol 812 de la société Hüls) | 0,8 |

La préparation de la lotion est réalisée en deux temps:
- On dissout d'abord le chlorure de sodium dans l'eau, puis on y ajoute les autres ingrédients de la phase aqueuse dans l'ordre en mettant en dernier le D-panthénol. On refroidit aux environs de -3°C la phase hydroalcoolique obtenue et on la filtre sur filtre-presse à cette température.
- On mélange par ailleurs tous les ingrédients de la phase huileuse dans l'ordre en agitant faiblement.
- On additionne ensuite les deux phases à température ambiante pour obtenir la lotion biphasique.

Il faut agiter la lotion avant chaque utilisation.

La demanderesse a trouvé ici un nouveau concept de produit parfumant biphasique à mélanger avant emploi, ayant un aspect transparent attractif. Ce produit peut attirer à lui une nouvelle clientèle à l'affût de la nouveauté dans le domaine de la cosmétique. Par ailleurs, son application est aussi fraîche que celle d'une eau de toilette classique, et son caractère irritant est bien moindre.

## Revendications

1. Composition liquide parfumante, caractérisée en ce qu'elle comporte deux phases distinctes, une phase hydroalcoolique contenant de 40 à 90 % d'éthanol et au moins une substance aromatique, et une phase huileuse contenant au moins une huile de silicone volatile.

2. Composition selon la revendication 1 caractérisée en ce qu'elle a un degré d'alcool inférieur à 60°.

3. Composition selon la revendication 1 ou 2, caractérisée en ce qu'elle contient un mélange d'huiles essentielles et/ou de substances odorantes.

4. Composition selon la revendication 3, caractérisée en ce que le mélange est un parfum.

5. Composition selon l'une quelconque des revendications précédentes, caractérisée en ce que la phase hydroalcoolique représente de 80 % à 20 % en poids par rapport au poids total de la composition et la phase huileuse représente de 20 % à 80 % en poids par rapport au poids total de la composition.

6. Composition selon l'une quelconque des revendications précédentes, caractérisée en ce que la phase hydroalcoolique contient au moins un adjuvant hydrosoluble.

7. Composition selon l'une quelconque des revendications précédentes, caractérisée en ce que la phase hydroalcoolique contient au moins un glycol.

8. Composition selon l'une quelconque des revendications précédentes, caractérisée en ce que l'huile de silicone volatile est choisie parmi les cyclométhicones ou les diméthicones.

9. Composition selon l'une quelconque des revendications précédentes, caractérisée en ce que l'huile de silicone volatile représente de 70 % à 80 % en poids par rapport au poids total de la phase huileuse.

10. Utilisation de la composition selon l'une quelconque des revendications précédentes comme eau de toilette.

11. Utilisation de la composition selon l'une quelconque des revendications 1 à 9 comme lotion après-rasage.

## Claims

1. Perfuming liquid composition, characterized in that it contains two distinct phases, an aqueous/alcoholic phase containing from 40 to 90 % of ethanol and at least one aromatic substance and an oily phase containing at least one volatile silicone oil.

2. Composition according to Claim 1, characterized in that its degree of alcohol is less than 60°.

3. Composition according to Claim 1 or 2, characterized in that it contains a mixture of essential oils and/or of scented substances.

4. Composition according to Claim 3, characterized in that the mixture is a fragrance.

5. Composition according to any one of the preceding claims, characterized in that the aqueous/alcoholic phase represents from 80 % to 20 % by weight with respect to the total weight of the composition and the oily phase represents from 20 % to 80 % by weight with respect to the total weight of the composition.

6. Composition according to any one of the preceding claims, characterized in that the aqueous/alcoholic phase contains at least one water-soluble adjuvant.

7. Composition according to any one of the preceding claims, characterized in that the aqueous/alcoholic phase contains at least one glycol.

8. Composition according to any one of the preceding claims, characterized in that the volatile silicone oil is chosen from cyclomethicones or dimethicones.

9. Composition according to any one of the preceding claims, characterized in that the volatile silicone oil represents from 70 % to 80 % by weight with respect to the total weight of the oily phase.

10. Use of the composition according to any one of the preceding claims as toilet water.

11. Use of the composition according to any one of Claims 1 to 9 as aftershave lotion.

## Patentansprüche

1. Flüssige parfümierende Zusammensetzung,
**dadurch gekennzeichnet, daß**
sie zwei unterschiedliche Phasen, eine wässerig-alkoholische Phase, die 40 bis 90 % Ethanol und mindestens eine aromatische Substanz enthält, und eine Ölphase, die mindestens ein flüchtiges Siliconöl enthält, aufweist.

2. Zusammensetzung nach Anspruch 1, dadurch gekennzeichnet, daß sie einen Alkoholgehalt von Weniger als 60 ° aufweist.

3. Zusammensetzung nach Anspruch 1 oder 2, dadurch gekennzeichnet, daß sie ein Gemisch aus etherischen Ölen und/oder Duftstoffen enthält.

4. Zusammensetzung nach Anspruch 3, dadurch gekennzeichnet, daß das Gemisch ein Parfum ist.

5. Zusammensetzung nach einem der vorhergehenden Ansprüche, dadurch gekennzeichnet, daß die wässerig-alkoholische Phase 80 bis 20 Gew.-%, bezogen auf das Gesamtgewicht der Zusammensetzung, und die Ölphase 20 bis 80 Gew.-%, bezogen auf das Gesamtgewicht der Zusammensetzung, ausmacht.

6. Zusammensetzung nach einem der vorhergehenden Ansprüche, dadurch gekennzeichnet, daß die wässerig-alkoholische Phase mindestens einen wasserlöslichen Zusatz enthält.

7. Zusammensetzung nach einem der vorhergehenden Ansprüche, dadurch gekennzeichnet, daß die wässerig-alkoholische Phase mindestens ein Glykol enthält.

8. Zusammensetzung nach einem der vorhergehenden Ansprüche, dadurch gekennzeichnet, daß das flüchtige Siliconöl unter den Cyclomethiconen oder den Dimethiconen ausgewählt ist.

9. Zusammensetzung nach einem der vorhergehenden Ansprüche, dadurch gekennzeichnet, daß das flüchtige Siliconöl in einem Mengenanteil von 70 bis 80 Gew.-%, bezogen auf das Gesamtgewicht der Ölphase, vorliegt.

10. Verwendung der Zusammensetzung nach einem der vorhergehenden Ansprüche als Eau de Toilette.

11. Verwendung der Zusammensetzung nach einem der Ansprüche 1 bis 9 als After-shave-Lotion.
